# EUROPÄISCHE PATENTANMELDUNG

(11) **EP 1 184 009 A1**
(43) Veröffentlichungstag der Anmeldung: **06.03.2002**
(21) Anmeldenummer: 01112437.7
(22) Anmeldetag: 22.05.2001
(51) Int. Cl.: A61F 2/18, A61F 11/04

(54) **Vorrichtung zum Ankoppeln am Steigbügel der Gehörknöchelchen**

(30) Priorität: 24.08.2000 DE 20014659 U
(71) Anmelder: Heinz Kurz GmbH Medizintechnik, 72144 Dusslingen (DE)
(72) Erfinder: Kurz, Heinz, 72144 Dusslingen (DE)
(74) Vertreter: Möbus, Rudolf, Dipl.-Ing.

(57) **Zusammenfassung**

Eine Vorrichtung zum Ankoppeln eines mindestens teilweise in das Mittelohr implantierbaren Hörgeräts oder einer passiven Gehörknöchelprothese am Steigbügel (11) der menschlichen Ossikelkette, die einen am Steigbügel (11) selbsthaltend befestigbaren Clip (12) aufweist.

## Beschreibung

Die Erfindung betrifft eine Vorrichtung zum Ankoppeln eines mindestens teilweise in das Mittelohr implantierbaren Hörgeräts oder einer passiven Gehörknöchelprothese am Steigbügel der menschlichen Ossikelkette.

Bisher werden sowohl aktive als auch passive Hörimplantate durch Befestigungselemente in Form einer Hülse an einem der Gehörknöchelchen befestigt und anschließend das Befestigungselement mittels eines Crimpwerkzeuges an dem Gehörknöchel dauerhaft befestigt. Eine solche Befestigungsmethode ist in der DE 197 38 587 C1 beschrieben. Diese bekannte Befestigungsmöglichkeit von Mittelohrprothesen oder Hörgeräten ist jedoch relativ aufwendig. Nach dem Aufschieben des hülsenförmigen Befestigungselements auf das Gehörknöchelchen muss ein Crimpwerkzeug exakt platziert werden, um eine lagegenaue und sichere Befestigung des Implantats zu bewirken.

Der vorliegenden Erfindung liegt die Aufgabe zugrunde, eine Befestigungsmöglichkeit für aktive und passive Hörimplantate zu schaffen, die einfach durchzuführen ist und einen sicheren Halt der Prothese gewährleistet.

Die Aufgabe wird mit einer Vorrichtung zum Ankoppeln eines mindestens teilweise in das Mittelohr implantierbaren Hörgeräts oder einer passiven Gehörknöchelprothese am Steigbügel der menschlichen Ossikelkette gelöst, die erfindungsgemäß einen am Steigbügel selbsthaltend befestigbaren Clip aufweist.

Mit Hilfe des Clips kann das aktive oder passive Hörimplantat ohne Zuhilfenahme von Werkzeugen rasch und dauerhaft am Steigbügel befestigt werden. Der Clip kann dazu Federzungen aufweisen, die axial über den Steigbügel schiebbar sind. Ein besonders sicherer Halt ist dabei gewährleistet, wenn der Clip den Steigbügel an vier Seiten umgreift.

Da die Größe und die relative Lage der Gehörknöchelchen hei jedem Mensch differiert, ist es vorteilhaft, wenn der Clip eine bewegliche Verbindung zum Hörgerät oder zur Gehörknöchelprothese aufweist. Es ist dann eine Anpassung der Prothese oder des Hörgeräts an die jeweiligen Gegebenheiten im Mittelohr in situ möglich. Die bewegliche Verbindung kann beispielsweise von einem Schaft aus einem flexiblen Material gebildet sein. Es ist jedoch auch möglich, zwischen dem Clip und dem Hörgerät oder der Prothese ein Kugelgelenk vorzusehen.

Insbesondere bei einer Verbindung mit einem flexiblen Schaft kann der Clip einteilig mit dem Hörgerät oder der Gehörknöchelprothese ausgebildet sein. Er bildet dann eines der Enden der Prothese oder des Hörgeräteimplantats.

Die Clipverbindung - sei es in Form eines flexiblen Schafts oder eines Kugelgelenks - kann jedoch auch mittels einer Steck- oder Klemmverbindung am Hörgerät oder der Gehörknöchelprothese befestigt werden.

Die gesamte Koppelvorrichtung kann aus einem biokompatiblen Material wie Titan oder einer Titanlegierung gefertigt sein, um Abstoßungsreaktionen des Körpers zu verhindern.

Nachfolgend werden bevorzugte Ausführungsformen einer erfindungsgemäßen Vorrichtung anhand der Zeichnung näher beschrieben.

### Im Einzelnen zeigen:

- Fig. 1: eine perspektivische Ansicht einer ersten Koppelvorrichtung;
- Fig. 2: eine perspektivische Ansicht einer zweiten Koppelvorrichtung;
- Fig. 3: eine perspektivische Ansicht einer dritten Koppelvorrichtung;
- Fig. 4: eine perspektivische Ansicht einer vierten Koppelvorrichtung;
- Fig. 5: eine perspektivische Ansicht einer einteilig mit einer Prothese ausgebildeten Koppelvorrichtung;
- Fig. 6: eine perspektivische Ansicht einer Koppelvorrichtung mit einer Gehörknöchelprothese.

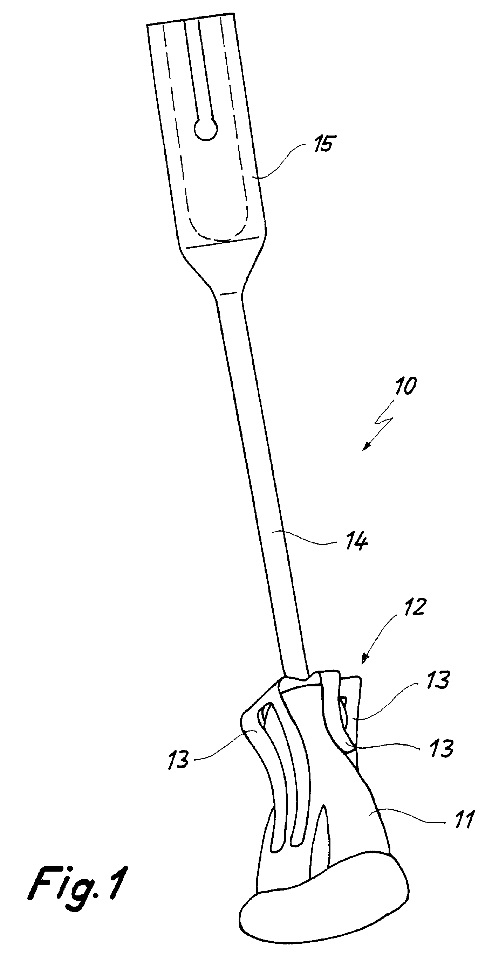

Fig. 1 zeigt eine erste Koppelvorrichtung 10 zur Ankopplung eines hier nicht dargestellten Hörimplantats an den Steigbügel 11 einer menschlichen Ossikelkette. Die Koppelvorrichtung 10 weist an ihrem unteren Ende einen Clip 12 auf, der mehrere federnde Zungen 13 aufweist, die in axialer Richtung über den Steigbügel 11 schiebbar sind und dann den Steigbügel 11 von vier Seiten umgreifen. An den Clip 12 schließt ein Schaft 14 aus einem flexiblen Material an, der in eine Steckhülse 15 übergeht, in der beispielsweise der Aktuator eines aktiven Hörimplantats oder aber auch der Schaft einer Gehörknöchelprothese verankerbar ist. Der flexible Schaft 14 ermöglicht eine Anpassung der Lage der gesamten Prothese je nach den anatomischen Gegebenheiten des Patienten.

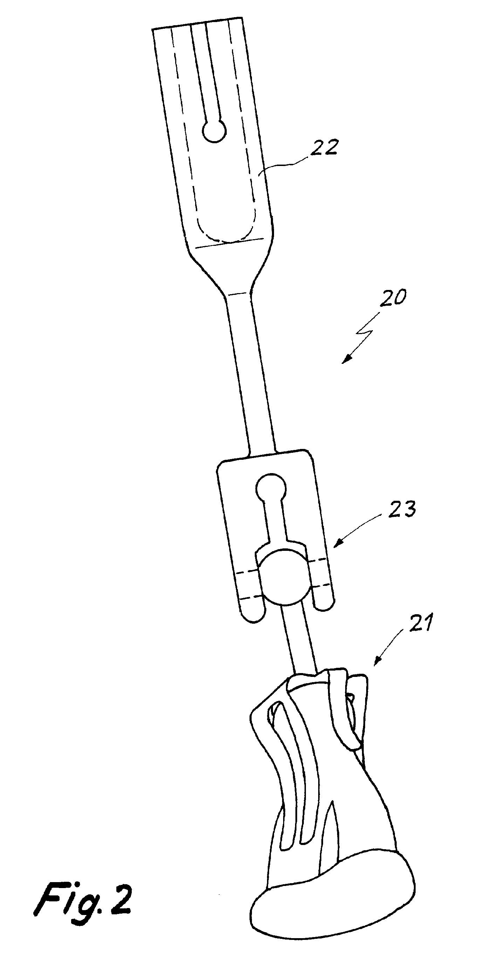

Die in Fig. 2 gezeigte Koppelvorrichtung 20 weist an ihrem unteren Ende ebenfalls wieder einen federnden Clip 21 gleicher Bauart wie der Clip 12 aus Fig. 1 auf. Auch die am oberen Ende angeordnete Steckhülse 22 entspricht in ihrem Aussehen der Hülse 15 aus Fig. 1. Gegenüber der Koppelvorrichtung 10 aus Fig. 1 unterscheidet sich die Koppelvorrichtung 20 durch die bewegliche Verbindung zwischen Hülse 22 und Clip 21, die hier nicht durch einen flexiblen Schaft sondern durch ein Kugelgelenk 23 gebildet wird.

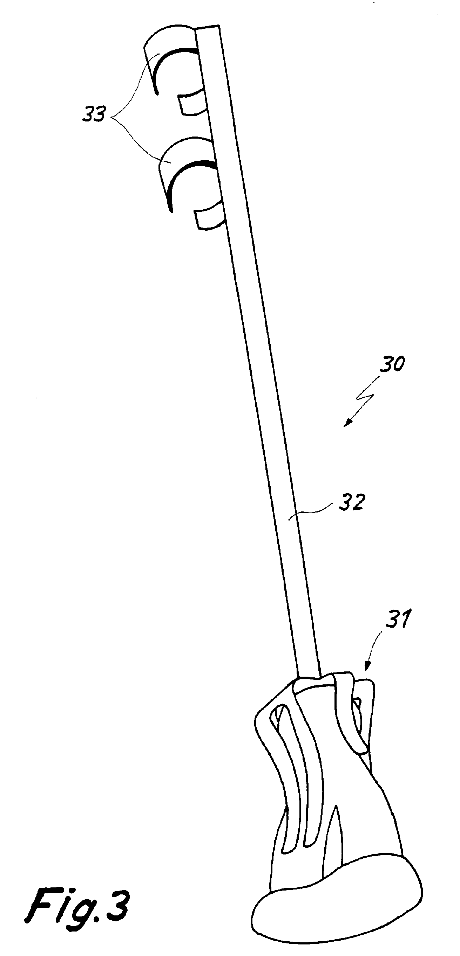

Fig. 3 zeigt eine Koppelvorrichtung 30, die Ähnlichkeit mit der Koppelvorrichtung 10 aus Fig. 1 aufweist. Es ist wieder ein federnder Clip 31 des gleichen Aufbaus wie der Clip 12 aus Fig. 1 vorgesehen. Daran schließt sich ein Schaft 32 aus einem flexiblen Material an. Bei der Koppelvorrichtung 30 erfolgt jedoch die Ankopplung an das Hörimplantat nicht über eine Steckhülse sondern über Klammern 33 und die Klemmwirkung dieser Klammern.

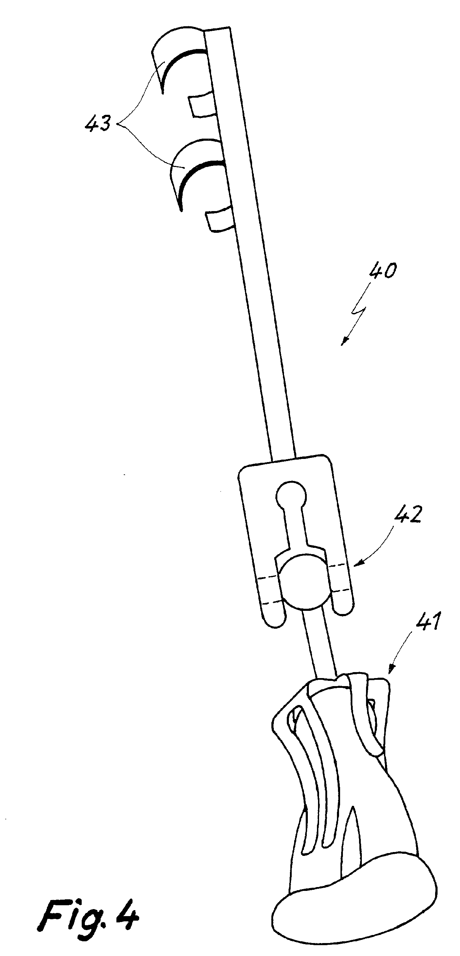

Fig. 4 zeigt eine der Fig. 3 entsprechende Koppelvorrichtung 40, bei der jedoch der flexible Schaft 32 durch ein Kugelgelenk 42 ersetzt ist. Die Befestigung der Koppelvorrichtung an der Prothese erfolgt auch hier wieder über Klammern 43.

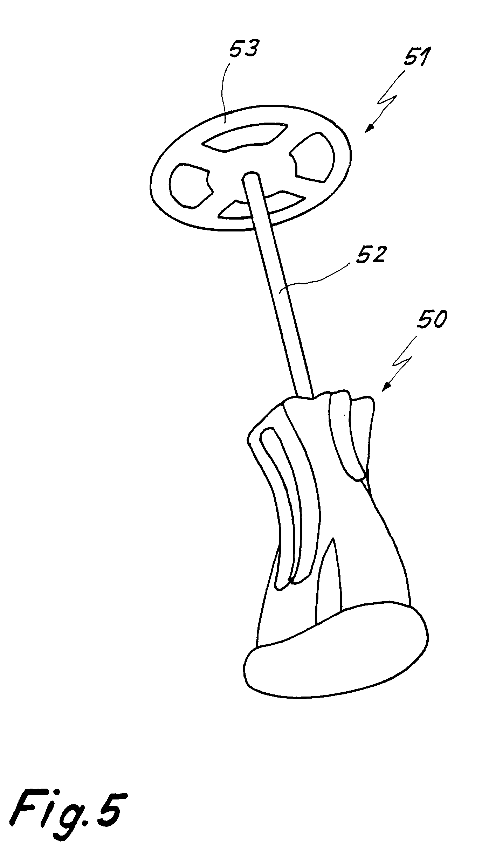

Fig. 5 zeigt nun eine Koppelvorrichtung 50 für eine Gehörknöchelprothese 41, die in diesem Fall streng genommen nur aus einem federnden Clip der gleichen Bauart wie der Clip 12 aus Fig. 1 besteht. Ein an den Clip 50 anschließender Schaft 52 bildet gleichzeitig den Schaft der Prothese 51 und kann vorzugsweise aus einem flexiblen Material gefertigt sein. Am gegenüberliegenden Ende des Schaftes 52 ist eine Gitterplatte 53 angeordnet, die in Anlage an das Trommelfell gebracht wird.

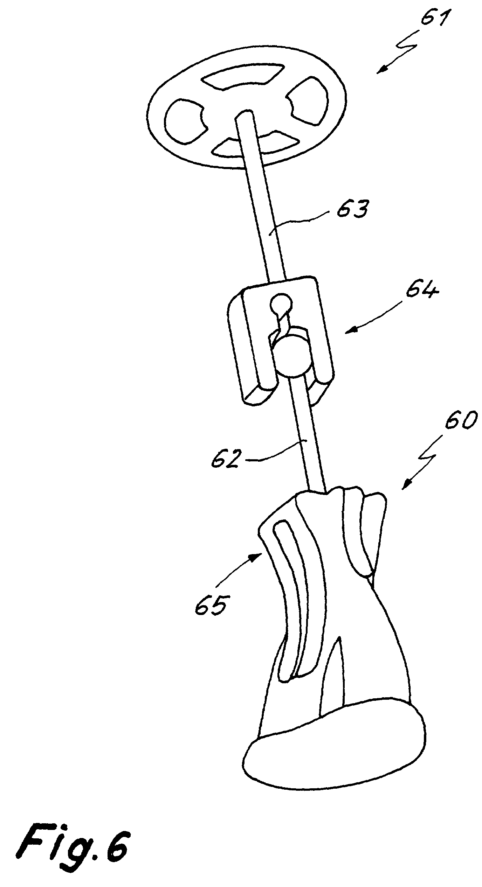

Eine ganz ähnliche Koppelvorrichtung 60 für eine Prothese 61 zeigt Fig. 6. Hier sind jedoch sowohl der Schaft 62 der Koppelvorrichtung 60 als auch der Schaft 63 der Prothese 61 aus einem steifen Material gefertigt. Um dennoch eine flexible Anpassung der Prothese 61 an die anatomischen Gegebenheiten des Patienten zu ermöglichen, ist zwischen den Schäften 62 und 63 ein Kugelgelenk 64 angeordnet.

## Patentansprüche

1. Vorrichtung zum Ankoppeln eines mindestens teilweise in das Mittelohr implantierbaren Hörgeräts oder einer passiven Gehörknöchelprothese (51, 61) am Steigbügel (11) der menschlichen Ossikelkette, **dadurch gekennzeichnet, dass** sie einen am Steigbügel (11) selbsthaltend befestigbaren Clip (12, 21, 31, 41, 50, 65) aufweist.

2. Vorrichtung nach Anspruch 1, **dadurch gekennzeichnet, dass** der Clip (12, 21, 31, 41, 50, 65) Federzungen (13) aufweist, die axial über den Steigbügel (11) schiebbar sind.

3. Vorrichtung nach Anspruch 1 oder 2, **dadurch gekennzeichnet, dass** der Clip (12, 21, 31, 41, 50, 65) den Steigbügel (11) an vier Seiten umgreift.

4. Vorrichtung nach einem der Ansprüche 1 bis 3, **dadurch gekennzeichnet, dass** der Clip (12, 21, 31, 41, 50, 65) eine bewegliche Verbindung zum Hörgerät oder zur Gehörknöchelprothese (51, 61) aufweist.

5. Vorrichtung nach Anspruch 4, **dadurch gekennzeichnet, dass** die bewegliche Verbindung von einem Schaft (14, 32, 52) aus einem flexiblen Material gebildet wird.

6. Vorrichtung nach Anspruch 4, **dadurch gekennzeichnet, dass** die bewegliche Verbindung von einem Kugelgelenk (23, 42, 64) gebildet wird.

7. Vorrichtung nach einem der Ansprüche 1 bis 6, **dadurch gekennzeichnet, dass** sie einteilig mit dem Hörgerät oder der Gehörknöchelprothese (51) ausgebildet ist.

8. Vorrichtung nach einem der Ansprüche 1 bis 6, **dadurch gekennzeichnet, dass** sie mittels einer Steck- oder Klemmverbindung (15, 22, 33, 43) am Hörgerät oder der Gehörknöchelprothese befestigbar ist.

9. Vorrichtung nach einem der Ansprüche 1 bis 8, **dadurch gekennzeichnet, dass** sie aus einem biokompatiblen Material, vorzugsweise aus Titan oder einer Titanlegierung, gefertigt ist.
